# EUROPÄISCHE PATENTSCHRIFT

(11) **EP 0 866 876 B1**
(45) Veröffentlichungstag und Bekanntmachung des Hinweises auf die Patenterteilung: **13.02.2002**
(21) Anmeldenummer: 96941047.1
(22) Anmeldetag: 28.11.1996
(51) Int. Cl.: C12P 21/02, C12N 15/72, C12N 1/21, C07K 16/28

(54) **VERFAHREN ZUR HERSTELLUNG VON REKOMBINANTEN PROTEINEN IN E. COLI MITTELS HOCHZELLDICHTE-FERMENTATION**
PROCESS FOR THE PREPARATION OF RECOMBINANT PROTEINS IN E.COLI BY HIGH CELL DENSITY FERMENTATION
PROCEDE DE PRODUCTION DE PROTEINES RECOMBINANTES DANS E.COLI PAR FERMENTATION A HAUTE DENSITE CELLULAIRE

(30) Priorität: 11.12.1995 EP 95119478
(43) Veröffentlichungstag der Anmeldung: 30.09.1998
(73) Patentinhaber: MERCK PATENT GmbH, 64293 Darmstadt (DE)
(72) Erfinder: STRITTMATTER, Wolfgang, D-64372 Ober-Ramstadt (DE); MATZKU, Siegfried, D-64673 Zwingenberg (DE); RIESENBERG, Dieter, D-07743 Jena (DE); HORN, Uwe, D-06567 Bad Frankenhausen (DE); KNÜPFER, Uwe, D-07747 Jena (DE); KUJAU, Marian, D-07747 Jena (DE); WENDEROTH, Rolf, D-07747 Jena (DE); PLÜCKTHUN, Andreas, CH-8006 Zürich (CH); KREBBER, Anke, CH-8006 Zürich (CH)
(86) Internationale Anmeldenummer: EP9605260
(87) Internationale Veröffentlichungsnummer: WO9721829

(56) Entgegenhaltungen:
- WO-A-92/15683
- BIO/TECHNOLOGY, Bd. 11, Nr. 11, 1.November 1993, Seiten 1271-1277, XP000608190 PACK P ET AL: "IMPROVED BIVALENT MINIANTIBODIES, WITH IDENTICAL AVIDITY AS WHOLE ANTIBODIES, PRODUCED BY HIGH CELL DENSITY FERMENTATION OF ESCHERICHIA COLI" in der Anmeldung erwähnt
- JOURNAL OF BIOTECHNOLOGY, Bd. 39, Nr. 1, 21.Februar 1995, Seiten 59-65, XP002026053 KORZ D. ET AL.: "Simple fed-batch technique for high cell density cultivation of Escherichia coli" in der Anmeldung erwähnt
- JOURNAL OF BIOTECHNOLOGY, Bd. 32, Nr. 3, 28.Februar 1994, Seiten 289-298, XP002026054 HELLMUTH K.: "Effect of growth rate on stability and gene expression of recombinant plasmids during continuous and high cell density cultivation of Escherichia coli TG1"
- BIO/TECHNOLOGY, Bd. 6, Dezember 1988, Seiten 1402-1405, XP002026055 GERDES K.: "The PARB (HOK/SOK) locus of plasmid R1: a general purpose plasmid stabilization system" in der Anmeldung erwähnt

## Beschreibung

Die Erfindung betrifft ein Fed-Batch Fermentationsverfahren mit speziellen Wirts-Vektor-Systemen von *E. coli* zur effektiven Bildung rekombinanter Proteine, insbesondere rekombinanter Antikörpermoleküle, insbesondere Andkörperfragmente, wie z.B. Miniantikörper.

Unter den erfindungsgemäßen Bedingungen können die *E. coli*-Zellen mit maximaler spezifischer Wachstumsrate bis zu sehr hohen Zelldichten wachsen. Nach Anschaltung der rekombinanten Produktbildung wirkt nur das gebildete Produkt begrenzend auf das Wachstum; eine Wachstumsbegrenzung durch Substrate oder metabolische Nebenprodukte erfolgt nicht. Auf diese Weise und in Verbindung mit den speziell hierfür angepaßten und hohe Stabilität aufweisenden neuen Expressionvektoren können hohe Raum-Zeit-Ausbeuten an rekombinanten Proteinen erzielt werden, die insbesondere im Fall von Antikörperfragmenten hohe biologische Aktivität aufweisen.

Eine wesentliche Voraussetzung für effektive rekombinante Proteinbildungen ist die Kultivierung von *E. coli -* Zellen zu hohen Zelldichten. Hierfür sind folgende Kultivierungen Stand der Verfahrenstechnik: Nach unlimitiertem Wachstum (µ = µₘₐₓ) in der Batch-Phase wird üblicherweise in der sich anschließenden Fed-Batch-Phase eine Kohlenstoffquelle (Glucose oder Glycerin) so begrenzt zudosiert, daß die Bildung von wachstumsinhibitorischen Nebenprodukten wie z.B. Acetat vermieden wird mit der Konsequenz, daß das Wachstum dann bis zum Erreichen hoher Zelldichten nur substratlimitiert (µ < µₘₐₓ) fortgesetzt werden kann (z.B. Riesenberg et al., 1991. J. Biotechnol., vol.20, 17-28; Strandberg et al. 1994. FEMS Microbiol. Rev., vol.14, 53-56; Korz et al. 1995. J. Biotechnol.39, 59-65; EP-B-0 511 226). Wachstum mit reduzierter Wachstumsrate hat natürlich lange Fermentationszeiten und somit folglich auch geringere Raum-Zeit-Ausbeuten zur Folge. Bei diesen Fermentationen ist aufgrund des sofortigen Verbrauchs die Konzentration der Kohlenstoffquelle in der Kulturlösung nahezu Null. Nach Anschaltung der rekombinanten Produktbildung ändert sich an den substratlimitierten Verhältnissen nichts.

Es sind auch Fed-Batch Kultivierungen mit E. coli bekannt, bei denen die Kohlenstoffquelle diskontinuierlich in größeren Zeitabständen und dann in größeren Mengen zugegeben wird, wobei meist ein Anstieg des pO₂-Wertes als Indikator für die Substraterschöpfung zur Initialisierung der folgenden Dosierung der C-Quelle verwendet wird (z.B. Eppstein et al. 1989. Biotechnol. 7, 1178-1181). Diese Verfahrensweise bedeutet einen häufigen Wechsel von längerfristigen Substratüberschußbedingungen zu Substratlimitbedingungen und impliziert somit metabolische Imbalanzen.

Im folgenden wird auf Fed-Batch Kultivierungen eingegangen, bei denen die Zellen in der Fed-Batch-Phase mit maximaler spezifischer Wachstumsrate (µ = µₘₐₓ) wachsen können. Fed-Batch Kultivierungen, bei denen nach off-line Bestimmungen größere Mengen an C-Quelle in größeren Zeitabständen der Kultur zur Vermeidung von Substratlimitationen zugegeben werden, sind experimentell aufwendig und haben den Nachteil, daß sich während der gesamten Fermentation die Konzentration der C-Quelle ständig ändert (z. B. Pack et al. 1993. Biotechnol., vol. 11, 1271-1277, Hahm et al. 1994. Appl. Microbiol. Biotechnol. 42, 100-107),

Es sind auch Fed-Batch Kultivierungen beschrieben, bei denen die Konzentration der C-Quelle on-line gemessen und geregelt wird, sodaß Limitationen vermieden werden, obwohl sie - insbesondere im Hochzelldichtebereich - mit den nachfolgend beschriebenen Nachteilen behaftet sind. Unlängst ist ein autoklavierbarer Glucose-Biosensor zum Einsatz, in mikrobiellen Fermentationen in Rührkesselfermentern beschrieben worden (M. R. Phelps et al. 1995. Biotechnol. Bioeng., vol. 46, 514-524). Er wurde für *E. coli*-Kulturen eingesetzt. Dieser in-situ-Sensor liefert in einer Zeitverzögerung von etwa 2 Minuten den aktuellen Wert in der Kulturlösung. Das vom Glucose-Sensor gelieferte Signal ist unter anderem pH- und pO₂-abhängig. Im Hochzelldichtebereich X > 80 g / l ist der Sensor nicht erprobt. Erfahrungsgemäß können Bewachsungen von in situ-Sonden mit *E. coli* bei sehr hohen Zelldichten zu weiteren Fehlwerten führen. Außerdem ist der Sensor während einer laufenden Fermentation nicht exakt rekalibrierbar. Andere Verfahren basieren nicht auf Messungen mit einem in situ-Sensor, sondern gründen sich z.B. auf der Bestimmung der Kohlenstoffquellen mittels on-line-Fließinjektions-Analysatoren (FIA) oder on-line-HPLC in zellfrei gemachter Kulturlösung, die aus dem Fermenter semikontinuierlich entnommen und einer Filtration oder Mikrozentifugation unterworfen wird (Kleman et al. 1991. Appl. Environ. Microbiol. 57, 910-917 und 918-923; Turner et al. 1994. Biotechnol. Bioeng. 44, 819-829). Vorhersage- und Rückkopplungs-Kontrollalgorithmen verringerten die Schwankungen der Glucosekonzentration beim Wachstum bis X= 65 g/l (Kleman et al. 1991. Appl. Environ. Microbiol. vol. 57, 910-917.). Im Bereich sehr hoher Zelldichten (ab ca. 80 g / l bis 150 g / l) wird die Separation von Zellen und Nährlösung zunehmend schwieriger und zeitaufwendiger, so daß auch die Zeitverzögerung zur Bestimmung des aktuellen Glucosewertes im Fermenter biomasseabhängig zunimmt und die Konstanthaltung des Glucosespiegels erschwert bzw. unmöglich macht. Mit konstanter und kurzer Zeitverzögerung dagegen wird die Glucosekonzentration gemessen bei einer Apparatur, die auf diese Zellseparation verzichtet (Pfaff et al. 1995. p. 6-11. In: Proceedings of the 6th International Conference on Computer Appl. in Biotechnol. Garmisch-Partenkirchen, FRG). Nach Pfaff et al. wird in unmittelbarer Nähe der Probennahmestelle nach Verdünnung der Kultur mit einem Wachstumsinhibitor eine FIA mit enzymatisch-amperiometrischem Glucose-sensor zum Einsatz gebracht .

Bei der aeroben Kultivierung bilden *E. coli*-Zellen, die nicht durch Dosierungsregime zum substratlimitierten Wachstum gezwungen werden, üblicherweise verstärkt das metabolische Nebenprodukt Acetat (Riesenberg 1991. Curr. Opinion Biotechnol., vol. 2, 380-384.), das sich in der Nährlösung akkumuliert und in größeren Mengen wachstumsinhibitorisch wirkt (Pan et al. 1987. Biotechnol. Lett., vol. 2, 89-94). Deshalb sind bisher diese Fed-Batch-Kultivierungen zu hohen Zelldichten nur mit besonderen E. coli-Stämmen möglich, deren Acetat-Akkumulation durch gezielte genetische Veränderungen reduziert wurde bei Tolerierung anderer damit verbundener Nachteile. Zu den Abkömmlingen von E.coli K12 gehören phosphotransacetylase-negative Mutanten (Bauer et al. 1990. Appl. Environ. Microbiol., vol. 56, 1296-1302; Hahm et al. 1994. Appl. Microbiol. Biotechnol., vol. 42, 100-107.), die jedoch in Glucose-Mineralsalzmedien stark im Wachstum reduziert sind. Phelps und Mitarbeiter (s.o.) verwendeten als Wirt den *E. coli* Stamm TOPP5 für die nicht substratlimitierte Kultivierung bis zu einer Biomasse von X= 85 g/l. Dieser E. coli Stamm, der offenbar nicht stark Acetat akkumuliert, ist jedoch kein K-12 Stamm. E. coli TOPP5 bildet Hämolysin und ist somit ein pathogener Stamm, der aus Sicherheitsgründen nicht als Wirt für die Bildung rekombinanter DNA-Produkte im Industriesektor in Frage kommt. Durch Transformation von E. coli-Zellen mit einem Plasmid, das ein Gen zur Codierung von Acetolactatsynthase (ALS) enhält, konnte durch gezielte Reorientierung des intermediären Stoffwechsels eine Reduktion der Acetatakkumulation erzielt werden (San et al. 1994. In: Ann-N-Y-Acad-Sci, vol.721, 257-267). Diese Verfahrensweise ist aber mit dem Nachteil behaftet, daß bei Verwendung eines ALS-codierenden Plasmides in Kombination mit einem zweiten, das "Produktions"-Gen tragenden Plasmids unter Hochzelldichtebedingungen üblicherweise Instabilitäten auftreten. Durch Plasmidinstabilitäten, die insbesondere bei der Kultivierung zu sehr hohen Zelldichten verstärkt auftreten, wird die Effizienz von rekombinanten Produktbildungen häufig verringert.

Antikörper oder Antikörperfragmente, wie Fab', F(ab')2, Miniantikörper oder single-chain Fv's, erlangen im zunehmenderen Ausmaß Bedeutung im medizinischen und biotechnischen Bereich. Unter Miniantikörper ist im folgenden erfindungsgemäß ein bivalenter oder bispezifischer über pseudo-Hinge-Region verknüpftes single-chain Fv-Fragment zu verstehen. Dabei kann es wichtig werden, wie zum Beispiel in der Krebstherapie, große Mengen an Antikörpern (etwa 1 g / Dosis) zur Verfügung zu stellen. In *E. coli* können nun besonders leicht und gut monovalente Antikörperfragmente oder Fusionsproteine von diesen, bzw. multimere oder multispezifische Varianten davon, hergestellt werden. Diese Fragmente bzw. Varianten weisen eine kleine Größe verbunden mit einer hohen spezifischen Bindungsfähigkeit auf. (z.B. Plückthun A., 1992, Immunol. Rev. 130, 151-188; Pack et al, 1995, J. Mol. Biol. 246, 28-34). Proteine und insbesondere Antikörper müssen aber richtig gefaltet sein, um biologisch und funktionell wirksam zu sein. Dieses Problem muß bei der Ausbeutebetrachtung von gebildetem Antikörperfragment pro Zelle in Zusammenhang mit der Zelldichte beachtet werden. Ferner spielt die Primärsequenz des Antikörpers eine wichtige Rolle bei der Bestimmung der Ausbeute *in vitro* und der Faltung *in vivo* (Knappik A. und Plückthun A., 1995, Protein Engin. 8, 81-89). So werden z.B. Fab-Fragmente als unlösliche cytoplasmatische oder periplasmatische Aggregate exprimiert und *in vitro* rückgefaltet. So wird über Ausbeuten von etwa 0.14 g / l bei niedriger Zelldichte (Condra et al., 1990, J. Biol. Chem. 265, 2292-2295) und bis etwa 1-2 g / l unlöslicher Antikörper bei mittlerer Zelldichte (Shibui et al, 1993, Appl. Microbiol. Biotechnol. 38, 770-775) berichtet. Auch bivalente Miniantikörper (Pack et al., 1993, Biotechnol. 11, 1993, 1271-1277) können in biologisch-fünktioneller Form in Ausbeuten von etwa 0.2 g / l in *E. coli* erhalten werden. Durchschnittlich sind bei diesen Ausbeuten ca. 5 - 45 % ordnungsgemäß rückgefaltet.

In den bekannten *E. coli*-Sytemen wird die Fremdprotein-Bildung in der Regel auf geeignete Weise nach Erreichen angemessener Zelldichten entsprechend dem Expressionssystem durch ein regulierbares Promotersystem angeschaltet. Hier sind beispielsweise zu nennen (i) der *araBAD* Promoter in Gegenwart des *AraC* Repressors (induzierbar durch Arabinose) (z.B. Better et al., 1993, Proc. Natl. Acad. Sci. (USA) 90, 457-461), (ii) der *phoA*-Promoter (induzierbar durch Phosphat-Entzug) (z.B. Carter et al, 1992, Biotechnol. 10, 163-167), und (iii) das *lac*-Promotersystem (induzierbar durch IPTG) (Pack et al, 1993, l.c.). Das *lac*-System, das in der Regel eine gute Expression bewirkt, hat aber den Nachteil, daß einerseits eine unerwünschte Grundexpression vor Induktion des Promoters und andrerseits eine Plasmidinstabilität nach Induktion mit I PTG zu beobachten ist.

In einer besonderen Ausführungsform der Erfindung wird ein spezieller Vektor (pHKK) beschrieben, der als Fremdgen Sequenzen enthält, die für Fragmente des murinen bzw. humaniersierten Antikörpers MAb 425 codieren. MAb 425 (ATCC HB 9629) ist ein muriner monoklonaler Antikörper, der aus der bekannte menschlichen A432 Krebszellinie (ATCC CRL 1555) isoliert wurde und an das Epitop des humanen epidermischen Wachstumsfaktor-Rezeptors (EGFR, ein Glycoprotein von etwa 170 kD) bindet unter Inhibierung der Bindung des natürlichen Liganden EGF. Es ist gezeigt worden, daß MAb 425 cyrotoxische Wirkung auf Turnorzellen hat, bzw. diese am Wachstum zu hindern vermag (Rodeck et al., *Cancer Res.* 1987. *47*: 3692). Aus der WO 92/15683 sind humanisierte sowie chimere Formen des MAb 425 bekannt, einschließlich der DNA- und Aminosäuresequenzen ihrer leichten und schweren Ketten.

Ziel der Erfindung war es nun, ein Verfahren zur Herstellung von Fremdproteinen, insbesondere Antikörperfragmenten, in rekombinanten *E. coli-* Zellen unter Hochzelldichte-Bedingungen (HCDC = High Cell Density Culture) mit hohen Raum-Zeit-Ausbeuten und ohne wesentliche Wachstumsbeeinträchtigung durch Substrate oder Metaboliten und ohne nennenswerte Plasmidverluste, bzw. Plasmidinstabilitäten unter Gewährleistung eines großen Prozentsatzes an wirksamer biologischer Aktivität (Bindungsfähigkeit, korrekte Faltung) des exprimierten Proteins zur Verfügung zu stellen.

Das erfindungsgemäße Verfahren ist ein mehrstufiges Batch-Verfahren, daß sich in erster Linie dadurch auszeichnet, daß die Zellen während des gesamten Batches maximal wachsen können (µ = µₘₐₓ). So können mit dem beschriebenen Verfahren letztlich Zelldichten von 100 bis 150 g / l (Biotrockenmasse) erreicht werden. Das Wachstum wird auch nicht wesentlich durch Acetat-Akkumulation inhibiert, da eine solche unter den gewählten Bedingungen überraschenderweise nicht besonders ausgeprägt ist, insbesondere bei Verwendung von E. Coli- Stämmen, die ohnedies nur zu verminderter Acetatbildung während der Fermentation neigen. Dies wird auch unter einer Reihe anderer zusätzlicher Maßnahmen vorallem dadurch erreicht, daß in der einer Batch-Phase nachgeschalteten Fed-Batch-Phase die Konzentration der Kohlenstoffquelle im Medium in einem definierten Bereich unter Beibehaltung von unlimitiertem Wachstum der Zellen konstant gehalten wird. Durch entsprechende Gestaltung des entsprechenden Expressionsvektors kann auch die unerwünschte Basalexpression von Protein vor Anschaltung der Proteinsynthese durch ein regulierbares Promotersystem nahezu ausgeschaltet werden, ebenso wie der zum Teil erhebliche Plasmidverlust, der, wie oben bereits erwähnt, normalerweise in Expressionssystemen mit starken Promotern wie z.B. das *lac*-Promotersystem, zu beobachten ist.

Es können durchschnittlich Proteinausbeuten von 3 bis 5 g / l nach ca. 25 bis 35 Stunden Gesamtkultivierung erreicht werden. Im Falle der wegen ihrer Faltungkskriterien besonders kritischen Antikörperfragmente, insbesondere Miniantikörper, sind etwa 80 % des synthetisierten Materials biologisch aktiv und korrekt gefaltet.

Gegenstand der Erfindung ist somit Verfahren zur Herstellung von Fremdprotein in *E. coli-* Zellen, die mit einem das Fremdgen und einen induzierbaren Promoter tragenden Plasmid transformiert wurden, mittels Hochzelldichtefermentation über Batch- und Fed-Batch Stufen ohne jegliche Wachstumsbegrenzung durch Substrate oder metabolische Nebenprodukte, und Isolierung und Aufreinigung des exprimierten Proteins aus dem Kulturmedium, wobei die Konzentration an Substraten in der Fed-Batch Phase über ein kontinuierliches automatisches oder semi-automatisches Analyse- und Zugabesystem gesteuert wird, wobei in der Fed-Batch Phase (i) die Konzentration der Kohlenstoffquelle im Medium in einem Bereich zwischen 0.1 g / l und 25 g / l unter Beibehaltung von unlimitiertem Wachstum der Zellen (µ = µₘₐₓ ) konstant gehalten wird, (ii) die Produktion des Fremdproteins innerhalb besagter Fed-Batch-Phase bei einer Zelldichte zwischen 10 und 80 g / l durch Induktion des Promoters gestartet wird, und (iii) nach erfolgter Produktsynthese-Induktion verwertbarer Stickstoff und Phosphat sowie Salze von Spurenelementen kontinuierlich zugefüttert werden, wobei (iv) während der gesamten Fed-Batch Phase der pO₂-Wert durch entsprechende Sauerstoffeinleitung in die Fermentationsbrühe zwischen 5 und 25 % eingestellt wird.

Die erfindungsgemäßen Werte für die erforderliche Konzentration der Kohlenstoffquelle während der Fed-Batch-Phase liegt in einem Bereich zwischen 0.1 g bis 25 g / l. Ein bevorzugter Bereich liegt zwischen 0.5 und 15 g / l, insbesondere zwischen 1.0 bis 5 g /l, bzw. zwischen 1.0 bis 3 g / l. Die besonders bevorzugte Konzentration ist 1.5 g / l. Als Kohlenstoffquelle sind vorzugsweise Glucose oder Glycerin oder Gemische aus beiden zu nennen. Erfindungsgemäß erfolgt die Zugabe der Kohlenstoffquelle mittels eines automatischen oder halbautomatischen Zugabe- und Analysesystems in kontinuierlicher Weise (on-line). Vorzugsweise wird ein on-line Fließinjektionsanalyse-System (FIA) eingesetzt.

Die Zufütterung von verwertbarem Stickstoff, vorzugsweise Ammonium-Stickstoff und Phosphat, beispielsweise Diammoniumhydrogenphosphat oder Ammoniumdihydrogenphosphat, sowie Spurenelementen, beispielsweise im Medium lösliche Salze von Bor, Mangan, Kupfer, Molybdän und Kobalt Eisen oder Zink, erfolgt in der der Batch-Phase nachgeschalteten Fed-Batch-Phase, vorzugsweise nach Anschaltung der Proteinsynthese durch den regulierbaren Promoter bei einer Zelldichte von 50 bis 80 g / l (Biotrockenmasse), vorzugsweise bei etwa 70 g / l bei einer Gesamtwachstumsrate von 100 bis 150, vorzugsweise 140 g / l.

Das Anschalten der Proteinsynthese durch Aktivierung des regulierbaren Promotersystems erfolgt erfindungsgemäß bei einer Zelldichte von 10 bis 80 g / l, vorzugsweise zwischen 20 bis 60 g / l; ganz besonders bevorzut ist der Bereich von 40 bis 50 g / l.

Der Partialsauerstoffdruck liegt während der Fed-Batch-Phase zwischen 5 und 25%, vorzugsweise zwischen 15 und 25%, ganz besonders bevorzugt bei 20 %.

Der pH-Wert des Fermentationsmediums ist erfindungsgemäß während des gesamten Batches zwischen 6.5 und 7.0, vorzugsweise zwischen 6.7 und 6.9, insbesondere bei 6.8, einzustellen.

Gegnstand der Erfindung ist ferner ein entsprechendes Verfahren bei dem ein Expressionsvektor mit einer von zwei Terminatorsequenzen flankierten das Fremgen enthaltenden Expressionskassette eingesetzt wird. Diese Terminator-Sequenzen, insbesondere die "upstream" positionierte, verhindern erfolgreich eine unerwünschte Expression von Protein vor der Anschaltung durch das Promotersystem. Besonders geeignet ist der Terminator tₕₚ (Nohno et al., 1988, J. Bacteriol. 170, 4097-4102) aber auch andere bekannte Terminator-Sequemzen können eingesetzt werden.

Weiterhin ist Gegenstand der Erfindung ein Verfahren, bei dem der verwendete Expressionsvektor zusätzlich ein Suizidsystem enthält. Das Suizidsystem produziert ein Protein, welches ohne das Vorhandensein des Plasmids in der Zelle für diese toxisch ist. Geeignete Suizidsysteme sind in der Literatur bekannt. Ein für die Erfindung besonders geeignetes Suizidsystem ist das *hok-sok-*System (z. B. Gerdes K., 1988, Biotechnol. 6, 1402-1405). Für das Verfahren zur effektiven Bildung rekombinanter Proteine, insbesondere von Antikörpermolekülen, ist es nämlich wichtig, daß das Wirts-Vektor-System sich im Hochzelldichtebereich durch eine hohe Plasmidstabilität, geringe rekombinante Basalexpression und hohe Produktbildung auszeichnet. Suizid-Systeme in Kombination mit rekombinanten, durch Terminatoren flankierten Expressionskassetten sind dabei vektorspezifisch.

Ferner ist Gegenstand der Erfindung ein entsprechendes Verfahren, bei dem ein Fremdgen eingesetzt wird, das für ein Antihörperfragment, insbesondere einen Miniantikörper codiert

Weiter ist Gegenstand der Erfindung ein Verfahren, bei dem Expressionsvektoren mit zusätzlichen, unten beschriebenen Merkmalen eingesetzt werden.

Prinzipiell sind die meisten bekannten, für die Rekombinationstechnik und für die Produktion im technischen Maßstab geeigneten *E. coli-* Stämme einsetzbar. Vorteilhafterweise finden solche Stämme bevorzugte Verwendung, die bei Wachstum zu hohen Zelldichten relativ wenig Acetat akkumulieren. Besonders geeignet sind Stämme, die eine Acetatanreicherung von unterhalb 5 g / l aufweisen. Überraschenderweise kann durch die gewählten Bedingungen des erfindungsgemäßen Verfahrens die Acetatakkumulation besonders tief gehalten werden. Besonders geeignet in dieser Hinsicht ist der allgemein bekannte und käuflich erhältliche *E. coli*-Stamm RV308 (A^{T}CC 31608) sowie seine wirkungsgleichen Varianten.

Gegenstand der Erfindung ist so insbesondere ein entsprechendes Verfahren, bei dem ein *E. coli*-Stamm mit einer Acetat-Akkumulation von unter 5 g / l im Kulturmedium während der Fermentation eingesetzt wird.

Gegenstand der Erfindung ist außerdem ein *E. coli*-Expressionsvektor, geeigenet für die Expression von Fremdproteinen unter Hochzelldichtefermentations-Bedingungen, der folgende Merkmale aufweist:
(i) eine "upstream"- und eine "downstream"- Terminatorsequenz,
(ii) das lac Promoter/Operatorsystem,
(iii) T7g10- Shine Delgarno-Sequenz,
(iv) die pelB- oder ompA-Signalsequenz,
(v) die Sequenz des Fremdgens,
und in einer bevorzugten Ausführungsform zusätzlich, ein Suizidsystem, insbesondere das *hok-sok-*Suizidsystem.

Erfindungsgemäß kann das Promotersystem auch durch andere geeignete, beispielsweise oben genannte Systeme ersetzt werden. Ebenso werden von der Erfindung auch andere gleichwirkende Signal-und Steuerungssequenzen umfaßt.

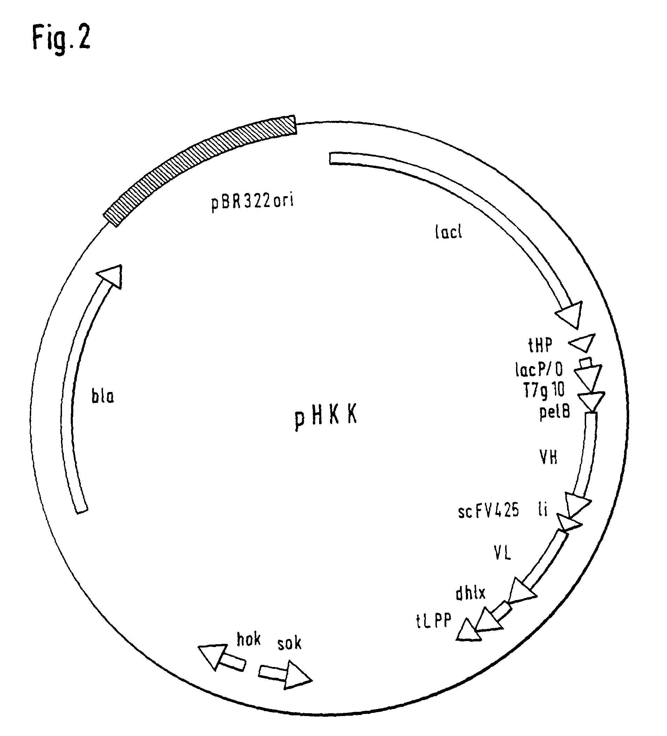

Als spezielle Ausführungsformen sind letztlich Gegenstand der Erfindung der durch seine Konstruktion definierte Expressionsvektor pHKK (Abb. 2), der die Sequenzen für den Miniantikörper, welcher sich aus MAb 425 ableitet, enthält, sowie ein spezieller rekombinater *E. coli*-Wirt RV308[pHKK].

### Beschreibung der Abbildungen:

- **Abb. 1:**: Experimenteller Versuchsaufbau des Bioreaktors zur Herstellung von Proteinen unter Hochzelldichte-Bedingungen. Das System ist mit einer Meß-, Anzeige-, Kontroll- und Dosierungsvorrichtung ausgestattet.
- **Abb. 2:**: Optimierter Expressionvektor pHKK sowie Teilstücke seiner Konstruktion. Der Vektor setzt sich im wesentlichen aus Teilstücken der bekannten Vektoren pASK40, pAK100 und pKG1022 zusammen.
- **Abb. 3(a-d):**: HCD-Kultivierung von rekombinanten *E. coli* am Beispiel von *E. coli* RV308[pHKK]: zeitlicher Verlauf von Biomasse, Glucose, Ammonium-Stickstoff, Phosphat, Acetat, Rührergeschwindigkeit, pO₂, O₂ und CO₂ im Abgas, Plasmidstabilität (ausgedrückt durch % von β-lactamase-positive Kolonien) und Bildung von Protein (hier: scFv₄₂₅dhlx). Die Batch- und Fed-Batch-Phasen sind in 5 Subphasen unterteilt. Der IPTG-Pfeil gibt den Start der Proteinproduktion an.

Das erfindungsgemäße Verfahren verwendet transformierte *E. coli-* Wirtszellen. Die ausgewählten Plasmidkonstruktionen richten sich nach der Art des zu exprimierenden Proteins. Besonders günstige Merkmale solcher Plasmidkonstruktionen werden weiter unten beschrieben. Die für die Plasmid-Konstruktion und die Wirtszellen-Transformation erforderlichen Techniken und Methoden sind allesamt bekannt und in der Literatur ausführlich beschrieben (z.B. Sambrook et al, 1989, Molecular Cloning: A Laboratory Manual, Cold Spring Harbor). Sie sind zudem anhand der besonderen Ausführungsformen der Erfindung in den Beispielen dargelegt. Ausgangsplasmide oder Plasmidteile sind entweder käuflich erhältlich oder können nach Standardmethoden auf Basis von bekannten Konstruktionsschemata ohne weiteres konstruiert werden.

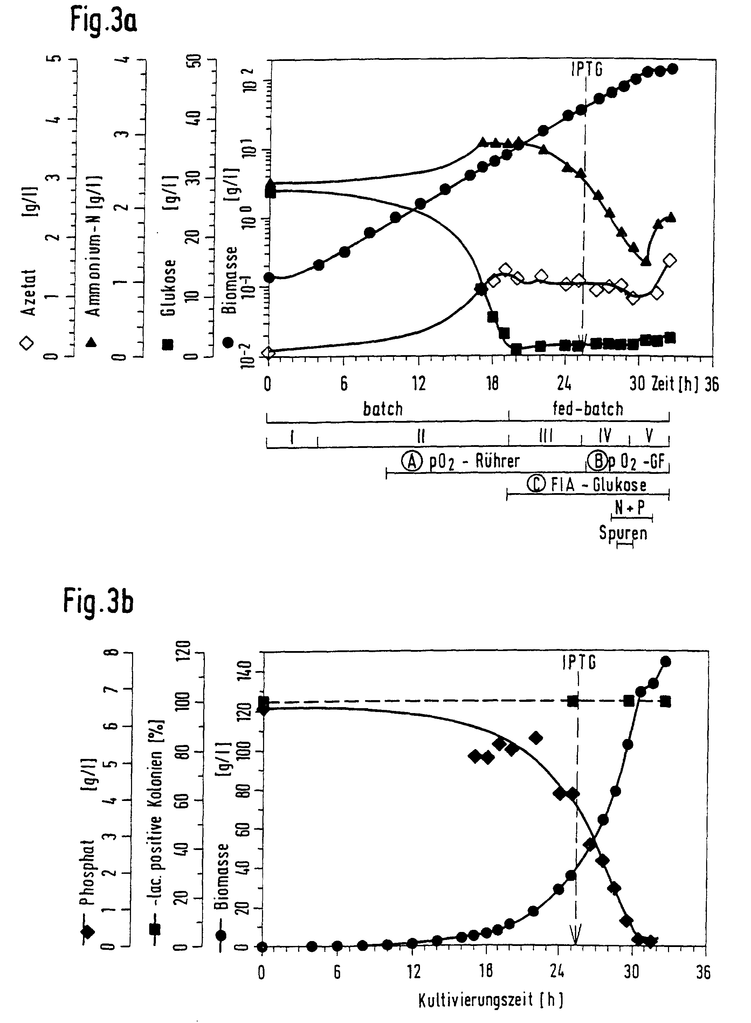

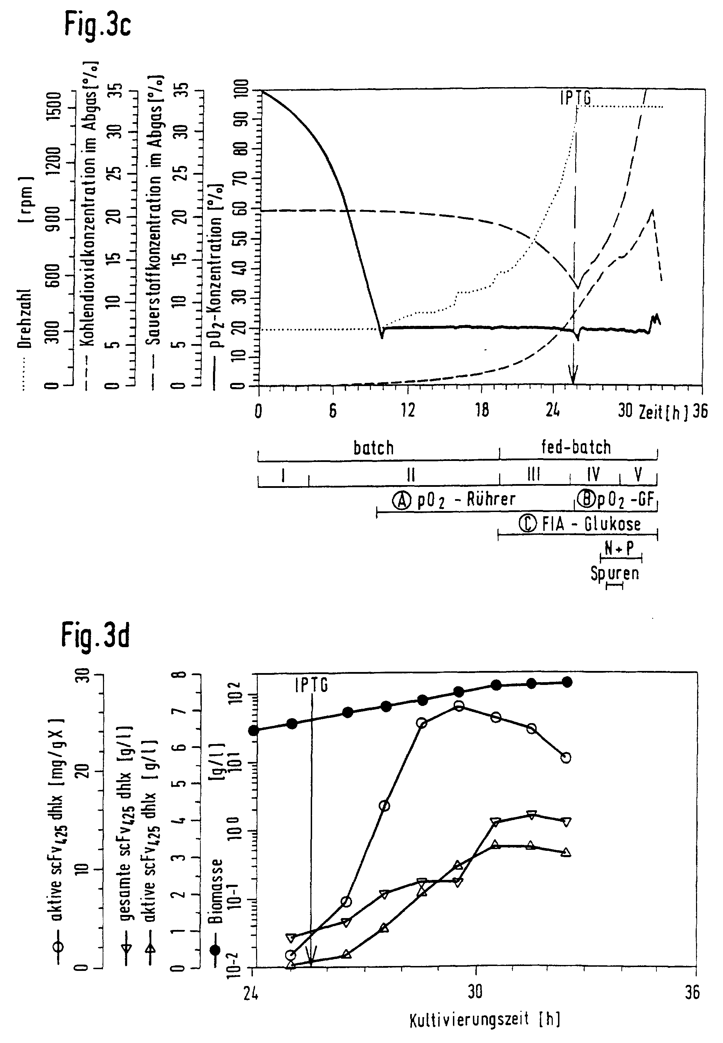

Die vorangeschaltete Batch-Phase einer typischen erfindungsgemäßen Fermentation von transformierten *E. coli* Zellen ist in zwei Subphasen unterteilt. Nach Animpfung mit einer entsprechenden Vorkultur ist Subphase I gekennzeichnet durch eine Lag-Phase, in der sich die Zellen adaptieren und die Wachstumsrate µ anschließend auf µₘₐₓ ansteigt. Während der Subphase II wachsen die Zellen exponentiell bei µ=µₘₐₓ. Nach einem Abfall von pO₂ von 100% Sättigung auf unter 5-15 % wird der pO₂-Wert durch Kontrollieren der Geschwindigkeit des pO₂-Rührers auf vorzugsweise auf pO₂-Werte zwischen 15 bis 25%, insbesondere um 20 % eingestellt (Abb. 3c). Diese Einstellung (durch Einleiten von mit reinem Sauerstoff angereicherter Luft) sollte etwa nach 6 bis 12 Stunden nach Beginn der Fermentation der Hauptkultur vorgenommen werden. Die Glucose-Konzentration, die anfänglich vorzugsweise zwischen 20 bis 35 g / l gelegen ist, sinkt bis zum Ende der Subphase II, welche auch das Ende der der Fed-Batch-Phase vorangeschalteten Batch-Phase darstellt, ab. Dabei sollten Werte von 0.1 g / l auf keinen Fall unterschritten werden. Dies wird von nun an verhindert durch entsprechendes Zufüttern von Glucose (Subphase III, Abb. 3a, Start der Fed-Batch-Phase). Erfindungsgemäß wird der Glucosewert zwischen 0.1 und 25 g / l, vorzugsweise aber zwischen 1 und 3 g / l konstant gehalten werden. Hierzu kann beispielsweise das Zufütterungsmedium FS1 (Tab. 1) verwendet werden. Da dieser Glucose-Konzentrationsbereich weit genug über dem Kₛ-Wert für Glucose liegt (Bergter, 1983, "Wachstum von Mikroorganismen", S. 41, Gustav Fischer Verlag, Jena), können die Zellen weiterhin bei µₘₐₓ wachsen. Die Kontrolle und Regelung der Glucosekonzentration erfolgt erfindungsgemäß mit einem automatischen oder halbautomatischen System.

Besonders geeignet sind Fließinjektionsanalysator-Syteme im on-line-Betrieb. Rein manuelle oder weitgehend manuelle Systeme haben sich als ungünstig erwiesen. Die Fed-Batch-Phase beginnt etwa zwischen der 15. und 22. Stunde, hängt aber letztlich von verschiedenen individuellen Faktoren wie Temperatur, Medienzusammensetzung und -Konzentration, Reaktorgröße usw. ab, insbesondere aber auch von der Art des verwendeten *E. coli*-Stammes. Vorteilhafterweise erfolgt etwa 4 bis 8 Stunden nach Beginn der Fed-Batch-Phase das Anschalten der Synthese des Fremdproteins. Der genaue Zeitpunkt richtet sich aber letztlich nach der zu diesem Zeitpunkt bereits erreichten Zelldichte der Kultur. Zelldichten zwischen 10 und 80 g / l, vorzugsweise zwischen 20 und 60 g / l sind besonders günstig, falls finale Zelldichten von 100 bis 150 g / l erreicht werden können. Allgemein günstig für das Starten der Proteinsynthese ist es somit, wenn etwa 10 bis 60 % der maximal zu erreichenden Zelldichte zum Induktionszeitpunkt vorliegen.

Die Proteinsynthese wird durcht Anschalten des regulierbaren Promotersystems bewirkt. Dieses Anschalten erfolgt je nach verwendetem System in der Regel durch Zugabe einer Substanz oder duch Veränderung einer physikalischen Größe. Im Falle des bevorzugten lac-Systems (Promoter, Operator, Induktor) durch Zugabe von IPTG (Isopropyl-thio-galactopyranosid). Das weitere Wachstum der Zellen ist jetzt nur noch durch das sich akkumulierende Produkt begrenzt. Deshalb ist es erfmdungsgemäß wichtig, daß vor Induktion keine nennenswerte Basalexpression stattfinden kann, die auf das Gesamtwachstum und damit auf die Gesamtausbeute einen ungünstigen Einfluß ausüben würde. Dies wird erfindungsgemäß dadurch bewerkstelligt, daß die Expressionskassette in dem Plasmid von wirksamen Terminatorsequenzen flankiert wird.

Ab dem Zeitpunkt der Glucose-Zufütterung verarmt das Fermentationsmedium an Stickstoff und Phosphat (Abb. 3a,b). Um Limitationen jeglicher Art zu vermeiden, wird Stickstoff und Phosphat vorzugsweise ebenfalls über ein entsprechendes kontinuierliches Verfahren zugefüttert. Stickstoff wird zweckmäßigerweise in Form von Ammoniumsalzen zugeführt, da hierdurch gleichzeitig auch auf den pH-Wert Einfluß genommen werden kann (6.5 bis 7.0, vorzugsweise 6.8). Beispielsweise eignet sich die Lösung FS2 (Tab. 1). Subphase IV ist gekennzeichnet durch erfindungsgemäßer Zuführung von Spurenelementen (z.B. Bor, Mangan, Eisen, Cobalt, Molybdän, Zinn) in Form ihrer löslichen Salze. Die Zugabe erfolgt in der Regel kontinuierlich mit einer konstanten Rate. Subphase V ist gekennzeichnet durch ein vermindertes Wachstum, in erster Linie bedingt durch Produktakkumulation. Außerdem ist ein geringfügiges Ansteigen der Acetatkonzentration zu beobachten. Dennoch ist die Acetatakkumulation bzw. -Konzentration im Medium überraschend niedrig. Dies ist auch auf die speziellen Verfahrensbedingungen zurückzuführen. *E. coli-*Stämme mit einer maximalen Acetatanreicherung von < 5 g / l während der Fermentation verstärken diesen Effekt noch deutlich.

Die Ausbeuten an Protein variieren je nach Art des Proteins durchschnittlich zwischen 2 und 6 g / l. Davon sind, wiederum je nach Art des Proteins, zwischen 50 und 95 % biologisch aktiv. Im Falle von Antikörperfragmenten erhält man über 80 % rückgefaltetes Protein. Diese Werte übersteigen deutlich die bisher im Stand der Technik beschriebenen vergleichbaren Verfahren.

Vorzugsweise können mit dem geschilderten Verfahren unter Einbezug der speziell hierfür konstruierten und angepaßten Expressionsplasmide Antikörperfragmente, insbesondere Miniantikörper, effektiv hergestellt werden. Aber auch die Herstellung vieler anderer Proteine, Fusionsproteine oder Enzyme ist gemäß des erfindungsgemäßen Verfahrens in vorteilhafter Weise möglich. Beispiele für solche geeigneten Proteine sind Hybridstreptokinase, Glucosedehydrogenase oder auch Proteine, die Wirkung auf die Blutgerinnung haben, wie z. B. Hirudin, Thrombin, Hementin oder Theromin.

**Tabelle 1:**

| Zusammensetzung der Medien; FS1, FS2 und FS3 stellen die Zufütterungs-Medien dar, die in den verschiedenen Subphasen der Fed-Batch-Phase verwendet werden. | | | | | | |
|---|---|---|---|---|---|---|
| | **Verbindung** | **Vorkultur-Medium mg/l** | **Haupt-Kultur-Medium mg/l** | **FS1 mg/l** | **FS2 mg/l** | **FS3 mg/l** |
| 1 | Na₂HPO₄ x 2H₂O | 8.6 x10³ | | | | |
| 2 | K₂HPO₄ | 3 x10³ | 16.6 x10³ | | | |
| 3 | (NH₄)₂HPO₄ | | 4 x10³ | | 227x10³ | |
| 4 | (NH₄)H₂PO₄ | | | | 169.5 x10³ | |
| 5 | NH₄Cl | 1 x10³ | | | | |
| 6 | NaCl | 5x10² | | | | |
| 7 | Zitronensäure | | 2.1 x10³ | | | |
| 8 | Fe(III)-citrat-hydrat | 60.0 | 75.0 | | | 5 x10³ |
| 9 | H₃BO₃ | 3.0 | 3.8 | | | 250 |
| 10 | MnCl₂ x 4H₂O | 15.0 | 18.8 | | | 125 |
| 11 | EDTA x 2H₂O | 8.4 | 10.5 | | | 700 |
| 12 | CuCl₂ x 2 H₂O | 1.5 | 1.9 | | | 125 |
| 13 | Na₂MO₄ x 2 H₂O | 2.5 | 3.1 | | | 213 |
| 14 | CoCl₂ x 6 H₂O | 2.5 | 3.1 | | | 213 |
| 15 | Zn(CH₃COO)₂x 2H₂O | 8.0 | 10 | | | 668 |
| 16 | Glucose | 10 x10³ | 25 x10³ | 670 x10³ | | |
| 17 | MgSO₄ x 7 H₂O | 600 | 1.5 x10³ | 19.8 x10³ | | |
| 18 | Ampicillin | 100 | 100 | | | |

### Beispiel 1:

Zur Herstellung eines rekombinanten *E. coli-*Wirts wurde der prototrophe *E. coli*-K12-Stamm RV308 (lac74-galISII::OP308strA), (Maurer et al., 1980, J. Mol. Biol. 139, 147-161; ATCC 31608) verwendet. Die Transformation mit einem zur Expression geeigneten Vektor sowie alle anderen erforderlichen DNA-Manipulationen erfolgten, sofern nicht anderswo erläutert, nach Standardmethoden. Plasmidfreie *E. coli* RV308 Zellen wurden als Kontrolle in einer entsprechenden Hochzelldichte-Fermentation eingesetzt.

Der Vektor pHKK wurde, wie folgt, konstruiert (Abb. 2): das kleine MluI-Fragment aus pAK100 (Krebber u. Plückthun, 1995), das den starken transkriptionalen Terminator t_{HP} (Nohno et al., s. o.) in der "upstream"- Region von lac p/o enthält, wurde in das Plasmid pASK40 (Skerra et al., 1991, Biotechnol. 9, 273-278) insertiert. Die Insertion von *hok-sok* DNA wurde durch zwei weitere Klonierungsschritte bewerkstelligt: das aphA-Gen aus pKG1022 (Gerdes, 1988, s. o.) wurde durch zweifache Verdauung mit XhoI und EcoRI entfernt, mit DNA Polymerase I (Klenow Fragment) aufgefüllt und religiert. In einem zweiten Schritt wurde das modifizierte BamHI-Fragment aus pKG1022 in die einzige BamHI-Schnittstelle des ersten Klonierungsproduktes kloniert. Der Miniantikörper stammt von einem single-chain Fragment ab, in dem die variablen Domänen in V_{H}-V_{L}-Richtung mit einem flexiblen Linker (gly₄ser)₃ verbunden wurden, gefolgt von einer prolinreichen Hinge-Region und einer abgewandelten "helix-turn-helix"- Domäne (dhlx) (Pack et al., 1993, Biotechnol. 11, 1271-1277). Die DNA-Sequenzen, Primer, Amplifikationen und Klonierungen der leichten und schweren Kette von murinem/humanisiertem MAb 425 ist in der WO 92/15683 ausführlich beschrieben. Zur Sekretion des scFv425dhlx-Fragmentes in das Periplasma wurde die V_{H}-Domäne N-terminal an die pelB-Signalsequenz fusioniert. Die T7g10-Ribosomen-Bindungsstelle (Shine Dalgarno) wurde mittels PCR-Methodik in die XbaI und SfiI -Schnittstellen von pEG1 (Strittmatter et al, 1995) kloniert. Die fertige scFv425dhlx Exprssionskassette wurde zwischen die XhaI und HindIII Schnittstellen kloniert. Hieraus ergab sich der Expressionsvektor pHKK gemäß Abb. 2.

### Beispiel 2:

Die Zusammensetzungen der Medien für die Vorkulturen in Erlenmeyer-Kolben, der Hauptkultur in einem mit Rührmechanik ausgestatteten Tankreaktor (Biostat ED10, B. Braun, Biotech International, Melsungen, FRG) sowie der Zufütterungsmedien FS1, FS2 und FS3 sind in Tabelle 1 zusammengestellt. Das Hauptkulturmedium (8 l) wurde im Vergleich zu dem Medium bei Riesenberg et al., 1991 (s. o.) modifiziert. Um Prezipitationen zu verhindern, wurden die Bestandteile in der angegebenen Reihenfolge der Tab. 1 hinzugegeben. Glucose und Magnesiumsulfat wurden als seperat autoklavierte Lösungen hinzugegeben. Der Reaktor wurde bei 26° C , einem Druck von 0.15 MPa, einem pH-Wert von 6.8 und einer durchschnittlichen Belüftungsrate von 10 l / min betrieben. Zur Einstellung des pH-Wertes wurde 25% -iger wäßriger Ammoniak verwendet. Ammoniak und Ucolub N115® (Fragol Industrieschmierstoffe GmbH, Mühleim/Ruhr, FRG) wurden mittels Sensorkontrolle während der Fermentation zur pH-Regulierung, bzw. als Antischaummittel hinzugegeben. FS1 wurde wie folgt, hergestellt: 750 g Glucose und 22.2 g MgSO₄ x 7H₂O wurden getrennt in 600 ml bzw. 50 ml H₂O gelöst. Die Lösungen wurden nach erfolgter Autoklavierung miteinander gemischt. FS2 wurde hergestellt durch Auflösen von 227 g (NH₄)₂HPO₄ und 169.5 g (NH₄)H₂PO₄ in Wasser unter Hinzufügung von 60 ml 25%-igem NH₃, um den pH-Wert auf 6.8 vor dem Autoklavieren einzustellen. FS3 wurde aus Stocklösungen in folgender Reihenfolge zubereitet: 50 ml Fe(III)-citrat-hydrat (6 g / l). 0.5 ml H₃BO₃ (30 g / l), 0.5 ml MnCl₂ x 4H₂O (10 g / l), 0.5 ml EDTA x 2H₂O (84 g / l), 0,5 ml CuCl₂ x 2H₂O (15 g / l), 0.5 ml Na₂MoO₄ x 2H₂O (25 g / l), 0,5 ml COCl₂ x 2H₂O (25 g / l) und 10 ml Zn(CH₃COO)₂ x 2H₂O (4 g / l).

### Beispiel 3:

Mehrere Kolonien aus einer Petrischale, gewachsen auf LB-Agar bei 26 °C, dienten dazu, 20 ml Flüssig-LB-Medium zu überimpfen. Nach 5-stündigem Schütteln (200 rpm, 26 °C) wurde 1ml in 100 ml Vorkulturmedium in einen 500 ml Kolben überführt und weiterinkubiert. 10 ml dieser Vorkultur wurden benutzt, um 100 ml neues Vorkulturmedium zu überimpfen. Auf diese Weise wurden 9 Vorkulturen erzeugt, die dazu benutzt wurden, zusammen 8 l Hauptkulturmedium im Fermenter mit einem anfänglichen OD₅₅₀ ≈ 0.2 zu überimpfen.

### Beispiel 4

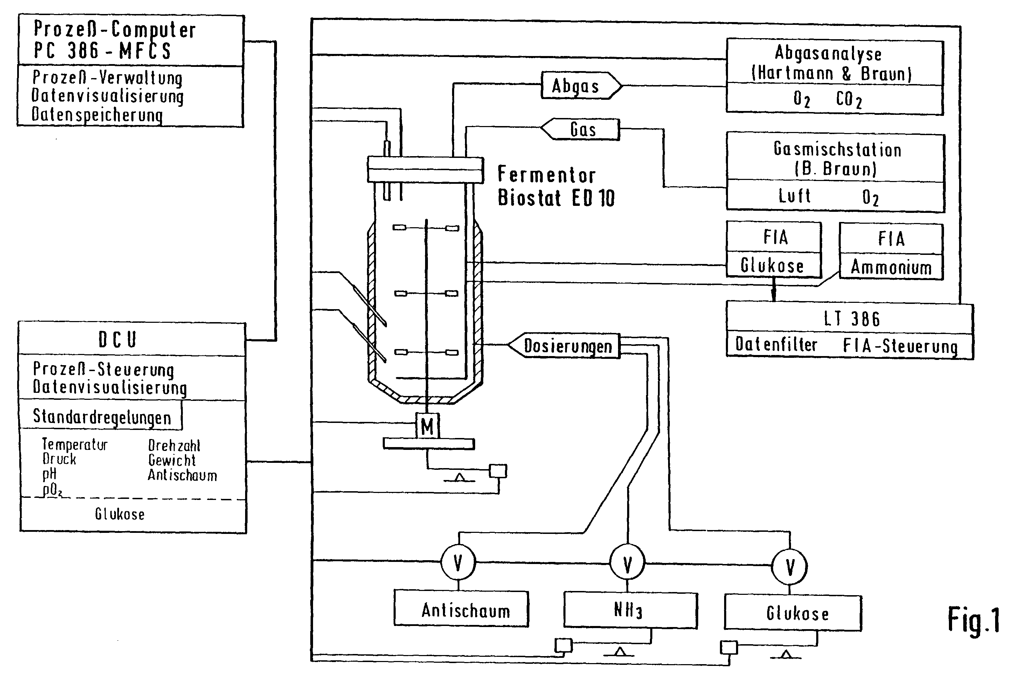

Der Aufbau des 10 I-Bioreaktors mit Zubehör und Kontrolleinrichtungen ist in Abb. 1 dargestellt. Die Kultivierung im Hochzelldichtefermentations-Maßstab erfolgte mittels einer digitalen Meß-und Kontrolleinheit (DCU), einem Multifermenter-Kontrollsystem (MFCS) und einer Gasfluß-Konrolleinheit. CO₂- und Sauerstoff-Abgabe wurden ständig gemessen. Nach Überimpfung diente der Bioprobensammler MX-3 (New Brunswick Scientific, Watford, UK), um aseptische Proben zu nehmen und off-line Datenanalyse zu ermöglichen (Webb et al., 1990, Biotechnol. 8, 926-928). Die Kontrolleinheiten hielten eine Einströmgasflußrate von 10 l / min, einen pH-Wert von 6.8, eine Temperatur von 26°C und einem Druck von 0.15 MPa aufrecht. Zwei Kontrollschleifen garantierten aerobe Wachstumsbedingungen bei einem pO₂-Wert von 20%. Alle wichtigen physikalischen Größen wurden während der ganzen Fermentation angezeigt und aufgezeichnet.

Während der Fed-Batch-Phase wird die Glucose-Konzentration in der Kultur bei 1.5 g / l gehalten. Hierzu wurde ein modifiziertes Flußinjektionsanalysegerät (FIAstar 5020 Analyzer mit Photometer und Detektionkontrolleinheit, Tecator AB, Schweden) eingesetzt. Details dieses Systems und seiner Arbeitsweise sind im Stand der Technik beschrieben (z. B. Pfaff et al., 1995, In Munack A., Schügerl K (eds.): Computer Applications in Biotechnology, Else vier Science Ltd., Oxford, 6-11).

Die Zelldichte wurde aus Messung der optischen Dichte bei 550 nm errechnet. Die Plasmidstabilität wurde nach Pack et al, 1993 (s. o.) bestimmt.

### Beispiel 5:

Die quantitative Bestimmung der synthetisierten Miniantikörper erfolgte nach der Methode von Pack et al., 1993 (s. o.). Die Menge der funktionsfähigen Miniantikörper wurde durch ELISA, die Gesamtmenge an Miniantikörper durch SDS-PAGE in 12 % Polyacrylamidgel nach Laemmli (1970) gefolgt von Gelscannen. Für die ELISAs wurden Mikrotiterplatten mit humanen EGFR-Rezeptor (z.B. aus WO 92/15683) beschichtet Die gebundenen Miniantikörper wurden mit anti-scFv425 Kanninchen-Serum und Peroxidase-konjugiertem Ziegen anti-Kanninchen IgG (Jackson Immunoresearch Inc, USA) detectiert. Die Ausbeute von aktiven Miniantikörpern wurde aus Verdünnungsreihen der aufgereinigten Miniantikörper errechnet. In einer Kontrolle wurde gezeigt, daß das anti-scFv425 Kanninchen-Serum keine feststellbare Kreuzreaktion mit anderen Komponenten des plasmidfreien Rohextraktes von *E. coli* RV308 aufweist. Ferner hatte der Zusatz von diesem Rohextrakt zu einer Verdünnungsreihe des gleichen Antikörpers in aufgereinigter Form keinen Effekt auf die ELISA-Signale. Zur Bestimmung der Gesamtmenge an Miniantikörper wurden Coomassie Brilliantblau gefärbte Gele photometrisch detektiert und die Konzentration der Miniantikörper wurde aus einer Verdünnungsreihe des aufgereinigten Miniantikörpers, der auf demselben Gel aufgetrennt worden war, errechnet. Als Kontrolle diente ein analoger Ansatz, bei dem *E. coli* Wirtszellen verwendet wurden, die keine Miniantikörper produzierten,

## Patentansprüche

1. Verfahren zur Herstellung von Fremdprotein in *E. coli-* Zellen, die mit einem das Fremdgen und einen induzierbaren Promoter tragenden Plasmid transformiert wurden, mittels Hochzelldichtefermentation über Batch- und Fed-Batch Stufen ohne jegliche Wachstumsbegrenzung durch Substrate oder metabolische Nebenprodukte, und Isolierung und Aufreinigung des exprimierten Proteins aus dem Kulturmedium, wobei die Konzentration an Substraten in der Fed-Batch Phase über ein kontinuierliches automatisches oder semi-automatisches Analyse- und Zugabesystem geregelt und / oder gesteuert wird, **dadurch gekennzeichnet, daß**
in der Fed-Batch Phase (i) die Konzentration der Kohlenstoffquelle im Medium in einem Bereich zwischen 0.1 g / l und 25 g / l unter Beibehaltung von unlimitiertem Wachstum der Zellen (µ = µₘₐₓ) konstant gehalten wird, (ii) die Produktion des Fremdproteins innerhalb besagter Fed-Batch Phase bei einer Zelldichte zwischen 10 und 80 g / l durch Induktion des Promoters gestartet wird, und (iii) nach erfolgter Produktsynthese-Induktion verwertbarer Stickstoff und Phosphat sowie Salze von Spurenelementen kontinuierlich zugefüttert werden, wobei (iv) während der gesamten Fed-Batch Phase der pO₂-Wert durch entsprechende Sauerstoffeinleitung in die Fermentationsbrühe zwischen 5 und 25 % eingestellt wird.

2. Verfahren nach Anspruch 1, **dadurch gekennzeichnet, daß** die Konzentration der Kohlenstoffquelle während der Fed-Batch Phase in einem Bereich zwischen 1 und 3 g / l konstant gehalten wird.

3. Verfahren nach Anspruch 1 oder 2, **dadurch gekennzeichnet, daß** Stickstoff, Phosphat und Spurenelemente bei einer Zelldichte zwischen 50 und 80 g / l zugesetzt werden.

4. Verfahren nach einem der Ansprüche 1 bis 3, **dadurch gekennzeichnet, daß** die Zelldichte gemaß (ii) aus Anspruch 1 20 bis 60 g / l beträgt.

5. Verfahren nach einem der Ansprüche 1 bis 4, **dadurch gekennzeichnet, daß** Zelldichten zwischen 100 und 150 g / l in der Fed-Batch Phase erreicht werden können.

6. Verfahren nach einem der Ansprüche 1 bis 5, **dadurch gekennzeichnet, daß** ein Expressionsvektor mit einer von zwei Terminatorsequenzen flankierten das Fremdgen enthaltenden Expressionskassette eingesetzt wird.

7. Verfahren nach Anspruch 6, **dadurch gekennzeichnet, daß** der verwendete Expressionsvektor zusätzlich ein Suizidsystem, vorzugsweise das *hok-sok*-Suizidsystem enthält.

8. Verfahren nach Anspruch 6 oder 7, **dadurch gekennzeichnet, daß** ein Fremdgen eingesetzt wird, das für ein Antikörperfragment, insbesondere einen Miniantikörper codiert.

9. Verfahren nach einem der Ansprüche 1 bis 4, **dadurch gekennzeichnet, daß** ein Expressionsvektor gemäß einem der Ansprüche 12 - 16 eingesetzt wird.

10. Verfahren nach einem der Ansprüche 1 bis 9, **dadurch gekennzeichnet, daß** ein *E. coli*-Stamm eingesetzt wird, der während der Fermentationsphase nicht mehr als 5 g / l Acetat im Kulturmedium akkumuliert.

11. Verfahren nach Anspruch 10, **dadurch gekennzeichnet, daß** der *E. coli*-Stamm RV308 (ATCC 31608) eingesetzt wird.

12. *E. coli*-Expressionsvektor, geeigenet für die Expression von Fremdproteinen unter Hochzelldichtefermentations-Bedingungen, **dadurch gekennzeichnet, daß** er folgende Merkmale enthält:
(i) eine upstream- und eine downstream-Terminatorsequenz,
(ii) das lac Promoter/Operatorsystem,
(iii) T7g10- Shine Dalgarno-Sequenz,
(iv) die pelB- oder ompA-Signalsequenz,
(v) die Sequenz des Fremdgens,

13. Vektor nach Anspruch 12, **dadurch gekennzeichnet, daß** er zusätzlich ein Suizid-Sytem, insbesondere das *hok-sok* Suizidsystem-Sequenz enthält.

14. Vektor nach Anspruch 12 oder 13, **dadurch gekennzeichnet, daß** die "upstream"- Terminatorsequenz t_{HP} und "downstream"-Terminatorsequenz t_{LPP} ist.

15. Vektor nach einem der Ansprüch 12 bis 14, **dadurch gekennzeichnet, daß** das Fremdgen eine Sequenz enthält, die für die V_{H}- und die V_{L}-Kette eines Miniantikörpers codiert.

16. Expressionsvektor mit der Bezeichnung pHKK gemäß dem Konstruktionsschema der Abb. 2.

17. Transformierter *E. coli*-Expressionswirt RV308[pHKK], erhältlich durch Transformation von RV308 (ATCC 31608) mit einem Expressionsvektor nach Anspruch 16.

## Claims

1. Process for preparing foreign protein in E. coli cells which have been transformed with a plasmid carrying the foreign gene and an inducible promoter, by means of high cell density fermentation by way of batch and fed-batch stages, without any restriction of growth by substrates or metabolic by-products, and isolation and purification of the expressed protein from the culture medium, with the concentration of substrates in the fed-batch phase being controlled using a continuous, automated or semi-automated analysis and addition system, **characterized in that**, in the fed-batch phase, (i) the concentration of the carbon source in the medium is kept constant in a range between 0.1 g/l and 25 g/l while maintaining unlimited growth of the cells (µ = µₘₐₓ), (ii) the production of the foreign protein is started in the said fed-batch phase by inducing the promoter at a cell density of between 10 and 80 g/l, and (iii) utilizable nitrogen and phosphate, and also salts of trace elements, are fed in continuously after induction of product synthesis has taken place, with (iv) the pO₂ being adjusted to between 5 and 25% during the whole of the fed-batch phase by passing oxygen into the fermentation broth in an appropriate manner.

2. Process according to Claim 1, **characterized in that** the concentration of the carbon source is kept constant in a range between 1 and 3 g/l during the fed-batch phase.

3. Process according to Claim 1 or 2, **characterized in that** nitrogen, phosphate and trace elements are added at a cell density of between 50 and 80 g/l.

4. Process according to one of Claims 1 to 3, **characterized in that** the cell density according to (ii) of Claim 1 is from 20 to 60 g/l.

5. Process according to one of Claims 1 to 4, **characterized in that** cell densities of between 100 and 150 g/l can be achieved in the fed-batch phase.

6. Process according to one of Claims 1 to 5, **characterized in that** an expression vector is employed which possesses an expression cassette which contains the foreign gene and is flanked by two terminator sequences.

7. Process according to Claim 6, **characterized in that** the expression vector which is used additionally contains a suicide system, preferably the hok-sok suicide system.

8. Process according to Claim 6 or 7, **characterized in that** a foreign gene is employed which encodes an antibody fragment, in particular a miniantibody.

9. Process according to one of Claims 1 to 4, **characterized in that** an expression vector according to one of Claims 12-16 is employed.

10. Process according to one of Claims 1 to 9, **characterized in that** an E. coli strain is employed which, during the fermentation phase, accumulates not more than 5 g/l acetate in the culture medium.

11. Process according to Claim 10, **characterized in that** the E. coli strain RV308 (ATCC 31608) is employed.

12. E. coli expression vector, suitable for expressing foreign proteins under high cell density fermentation conditions, **characterized in that** it contains the following features:
(i) an upstream terminator sequence and a downstream terminator sequence,
(ii) the lac promoter/operator system,
(iii) a T7g10 Shine Dalgarno sequence,
(iv) the pe1B or ompA signal sequence, and
(v) the sequence of the foreign gene.

13. Vector according to Claim 12, **characterized in that** it additionally contains a suicide system, in particular the hok-sok suicide system sequence.

14. Vector according to Claim 12 or 13, **characterized in that** the upstream terminator sequence is t_{HP} and the downstream terminator sequence is t_{LPP}.

15. Vector according to one of Claims 12 to 14, **characterized in that** the foreign gene contains a sequence which encodes the V_{H} chain and the V_{L} chain of a miniantibody.

16. Expression vector having the designation pHKK according to the construction scheme depicted in Fig. 2.

17. Transformed E. coli expression host RV308[pHKK], which can be obtained by transforming RV308 (ATCC 31608) with an expression vector according to Claim 16.

## Revendications

1. Procédé pour la production d'une protéine étrangère dans des cellules de *E. coli* qui ont été transformées par un plasmide portant le gène étranger et un promoteur inductible, par fermentation à haute densité cellulaire en stades en mode discontinu (*batch*) et en mode discontinu avec additions (*fed-batch*), sans aucune limitation de croissance par des substrats ou des sous-produits métaboliques, et isolement et purification de la protéine exprimée à partir du milieu de culture, la concentration de substrats dans la phase discontinue avec additions étant réglée et/ou commandée par un système d'analyse et d'addition en continu automatique ou semi-automatique, **caractérisé en ce que** dans la phase discontinue avec additions (I) la concentration de la source de carbone dans le milieu est maintenue constante dans une plage comprise entre 0,1 g/l et 25 g/l avec maintien d'une croissance illimitée des cellules (µ = µₘₐₓ), (Il) la production de la protéine étrangère dans ladite phase discontinue avec additions est déclenchée, à une densité cellulaire comprise entre 10 et 80 g/l, par induction du promoteur, et (III) une fois effectuée l'induction de la synthèse du produit, on ajoute en continu de l'azote et du phosphate utilisables ainsi que des sels d'oligo-éléments, (IV) pendant toute la durée de la phase discontinue avec additions la valeur de la pression d'oxygène pO₂ étant maintenue entre 5 et 25 % par introduction correspondante d'oxygène dans le bouillon de fermentation.

2. Procédé selon la revendication 1, **caractérisé en ce que** pendant la phase discontinue avec additions la concentration de la source de carbone est maintenue constante dans une plage comprise entre 1 et 3 g/l.

3. Procédé selon la revendication 1 ou 2, **caractérisé en ce qu'**azote, phosphate et oligo-éléments sont ajoutés à une densité cellulaire comprise entre 50 et 80 g/l.

4. Procédé selon l'une quelconque des revendications 1 à 3, **caractérisé en ce que** la densité cellulaire selon (II) de la revendication 1 va de 20 à 60 g/l.

5. Procédé selon l'une quelconque des revendications 1 à 4, **caractérisé en ce que** des densités cellulaires comprises entre 100 et 150 g/l sont atteintes dans la phase discontinue avec additions.

6. Procédé selon l'une quelconque des revendications 1 à 5, **caractérisé en ce qu'**on utilise un vecteur d'expression comportant une cassette d'expression contenant le gène étranger encadré de deux séquences de terminateur.

7. Procédé selon la revendication 6, **caractérisé en ce que** le vecteur d'expression utilisé contient en outre un système suicidaire, de préférence le système suicidaire *hok-sok.*

8. Procédé selon la revendication 6 ou 7, **caractérisé en ce qu'**on utilise un gène étranger qui code pour un fragment d'anticorps, en particulier un mini-anticorps.

9. Procédé selon l'une quelconque des revendications 1 à 4, **caractérisé en ce qu'**on utilise un vecteur d'expression selon l'une quelconque des revendications 12 à 16.

10. Procédé selon l'une quelconque des revendications 1 à 9, **caractérisé en ce qu'**on utilise une souche de *E. coli* qui n'accumule pas plus de 5 g/l d'acétate dans le milieu de culture pendant la phase de fermentation.

11. Procédé selon la revendication 10, **caractérisé en ce qu'**on utilise la souche de *E. coli* RV308 (ATCC 31608).

12. Vecteur d'expression dans *E. coli*, approprié pour l'expression de protéines étrangères dans des conditions de fermentation à haute densité cellulaire, **caractérisé en ce qu'**il comporte les caractéristiques suivantes :
(I) une séquence de terminateur en amont et une en aval,
(II) le système promoteur/opérateur lac,
(III) la séquence de Shine-Dalgàrno T7g10,
(IV) la séquence signal pelB ou ompA,
(V) la séquence du gène étranger.

13. Vecteur selon la revendication 12, **caractérisé en ce qu'**il contient en outre un système suicidaire, en particulier la séquence de système suicidaire *hok-sok*.

14. Vecteur selon la revendication 13 ou 14, **caractérisé en ce que** la séquence de terminateur "en amont" est t_{HP} et la séquence de terminateur "en aval" est t_{LPP}.

15. Vecteur selon l'une quelconque des revendications 12 à 14, **caractérisé en ce que** le gène étranger contient une séquence qui code pour la chaîne V_{H} et la chaîne V_{L} d'un mini-anticorps.

16. Vecteur d'expression portant la désignation PHKK, selon le schéma de construction de la figure 2.

17. Hôte d'expression transformé *E. coli* RV308[pHKK], pouvant être obtenu par transformation de RV308 (ATCC 31608) par un vecteur d'expression selon la revendication 16.
